# EUROPEAN PATENT APPLICATION

(11) **EP 3 384 904 A1**
(43) Date of publication of application: **10.10.2018**
(21) Application number: 17164658.1
(22) Date of filing: 03.04.2017
(51) Int. Cl.: A61K 9/70

(54) **PHARMACEUTICAL PREPARATION FOR DERMAL ADMINISTRATION**

(71) Applicant: tesa Labtec GmbH, 40764 Langenfeld (DE)
(72) Inventor: BREITENBACH,Armin, 51371 Leverkusen (DE); KERSKI, Sebastian, 42119 Wuppertal (DE)
(74) Representative: von Renesse, Dorothea

(57) **Abstract**

The invention pertains to a pharmaceutical preparation comprising an active pharmaceutical ingredient (API) and a film for use in the prophylactic or therapeutic treatment of a subject by dermal administration, wherein the API is deposited onto the film.

## Description

### FIELD OF THE INVENTION

The invention relates to the field of pharmaceutical preparations for use in the prophylactic or therapeutic treatment of a subject by dermal administration of an active pharmaceutical ingredient.

### BACKGROUND OF THE INVENTION

Macromolecules, in particular biopolymers, are of utmost importance in modern medicine as active pharmaceutical ingredients (API), for example as vaccines, allergens, antibodies and other proteinaceous agents. Macromolecules are typically applied to a subject by injection. Macromolecules which are to be injected can be stored in solution, which usually requires a cold chain to remain intact, or lyophilized and reconstituted immediately prior injection, which requires a specifically adapted lyophilization protocol and sterile handling upon reconstitution.

Another route of drug administration is the intradermal or transdermal route. Dermal administrations, i.e. via the intradermal or transdermal route, allow the drug to be administered directly at the site of the desired pharmacological action.

One approach to facilitate dermal delivery of macromolecules is through microporated skin. WO 2016/169971 A1 describes a vaccine preparation comprising an antigen for use in the prophylactic or therapeutic treatment of a subject by intradermal administration through laser-generated micropores. The preparation can be applied in the form of a transdermal patch.

Manufacturing dermal patches is often done by solvent casting or extrusion techniques. These methods typically involve forming a solution comprising the API and the film-forming substance, casting and drying the solution. During this process, the API is subjected to significant thermal stress which is even higher the more water is removed. Water removal is a particular challenge for the API's stability in the context of manufacturing dermal patches.

Therefore, it is an object of the present invention to provide an improved pharmaceutical preparation, which is suited for dermal administration of APIs, in particular of thermolabile biopolymers, such as proteins and nuclei acids.

### SUMMARY OF THE INVENTION

This object is solved by the pharmaceutical preparation according to claim 1, namely by a pharmaceutical preparation comprising an active pharmaceutical ingredient (API) and a film for use in the prophylactic or therapeutic treatment of a subject by dermal administration, wherein the API is deposited onto said film.

The API can be deposited onto the film by liquid deposition or solid deposition. Liquid deposition may be performed by printing the API onto the film. All types of printing methods can be applied. Solid deposition means that at least parts of the API are deposited on the film as a solid, e.g. in the lyophilized state.

Liquid deposition, such as printing, of an API-containing composition (hereinafter also called "ink") requires only little water. It is possible to adjust the residual water content of the pharmaceutical preparation to desired low levels because it is possible to vary the amount of water or solvent needed depending on the API and use only as little water or solvent as necessary. For solid deposition, it is even possible to mostly avoid water or solvent.

Thermal stress, such as e.g. the drying step above ambient temperature at the end of the solvent casting or extrusion method, can thus be reduced or even completely avoided. The API hence can remain in its native and functional state. Preferably, no drying at temperatures above ambient temperature (elevated temperatures, i.e. 30°C or more) step is required at all.

Many macromolecules are instable in water and/or instable when subjected to elevated temperatures. Accordingly, the pharmaceutical preparation of the present is in particular useful for the administration of APIs which are sensitive against thermal stress (thermolabile), e.g. such as in particular of high molecular weight biopolymers, most preferred of proteins.

The manufacture of the film by depositing the API can also be accomplished by a 3-D printing process. This prcoess als allows adjusting the residual water content to low levels and avoiding thermal stress. This is due to the stepwise approach of 3-D printing techniques that allow each printed layer to dry first at ambient temperature before the next layer is added. Thus, drying at elevated temperatures and thereby applying thermal stress to the API can be reduced, preferably fully avoided. The film prefrably dries during the printing process.

Reduction of thermal stress is especially advantageous when the pharmaceutical preparation of the invention includes a macromolecular API, in particular a biopolymer. These APIs are typically characterized by a high molecular weight, a good solubility in water and a relatively low stability. Due to the possibility to achieve low residual water content of the film with little or no drying of the film at temperatures above ambient temperature, preferably no such drying at all, a pharmaceutical preparation can be provided which can be stored for a long term at ambient temperature. The pharmaceutical composition thus is characterized by a long shelf life at room temperature; preferably at least 12 months, even more preferred at least 18 months.

Hence, a particular advantage of the invention is that there is no need for a cold chain for the further handling or storage of the pharmaceutical preparation. This is in particular advantageous if the API is a vaccine and the pharmaceutical preparation is used for vaccination.

Furthermore, the stability of the API in the pharmaceutical preparation allows minimizing the amount of preservatives. In an especially advantageous embodiment of the invention, the pharmaceutical preparation is even free of preservatives.

The API used according to the present invention preferably belongs to the group consisting of macromolecules (i.e. molecules with a molecular weight of at least 500 Da), preferably biopolymers, in particular vaccines, allergens and systemic drugs. Most preferred API is a thermolabile protein or nucleic acis with a molecular weight between 1000 and 400,000, preferably 10,000 to 200,000 Da.

In one particular embodiment of the invention the film is free of any API before the first layer of API is deposited onto the film. Such a film free of API is also called "placebo film". Nevertheless, according to the invention, an API which is sufficiently resistant against thermal stress can also be present in the film forming-composition during the manufacture of the film. A further API can be deposited onto the film after manufacture of that film. Hence in one embodiment of the invention the film is free of a thermolabile API, but can contain a thermostable API. A thermolabile API is then deposited onto the film.

A further advantage of the pharmaceutical preparation of the invention is that the waste of API during production can be reduced, preferably fully avoided, because only that amount of API is used which forms part of the later market product. The deposition allows to localize the API in distinct areas of the film. In contrast, for solvent casting or extrusion typically the API containing film is produced in ecxcess and then individual pieces thereof are punched out to provide the final market product. The API containing film produced in excess is then discarded.

With dispositing the API batch sizes of the pharmaceutical preparation are also more flexible and even individual dosing is possible.

Another advantage of depositing an API is that also low doses - typical for therapeutically relevant biopolymers - can be produced with highest precision exceeding the precision achieved with classical film manufacturing techniques. Furthermore, compared to e.g. solvent casting requiring a large floor space for the manufacturing equipment, the manufacturing by depositing can be performed even with low floor space requirement. This is advantageous especially when handling high containment drugs. Then not the entire production facility (or huge parts thereof) has to be contained, but only the rather small space around the depositing machine (printer).

It was found that the pharmaceutical preparation of the invention is in particular suitable for the dermal administration of APIs. This holds in particular true when the pharmaceutical preparation is applied on pre-treated skin. The APIs - and even macromolecular biopolymers such as proteins - can be absorbed through the dermis. This finding was surprising since the mobility of APIs, especially in case of macromolecules, was expected to be too low; in particular when the residual water content of the pharmaceutical preparation is below 15% by weight.

Without being bound by theory, it is believed that leakage of wound secretion from pre-treated skin or transpiration of the skin (i.e. transepidermal water loss) leads at least partly to the dissolution of the pharmaceutical preparation and thereby to the solubilization and furthermore to the mobilization of the APIs from the film into the skin. The invention does therefore not only simplify the storage of pharmaceutical preparations containing thermolabile API, but also provides a pharmaceutical preparation which improves the dermal administration of APIs.

The leakage of wound secretion is enhanced when the pharmaceutical preparation of the invention includes a backing, in particular an occlusive backing, most preferred a occlusive backing which does not allow water vapor to pass. Hence in a preferred embodiment of the invention the pharmaceutical preparation comprises a film, an API deposited thereon and furthermore a backing. The backing preferably is attached to the surface of the film turning away from the skin.

The pharmaceutical preparations of the invention are useful for the prophylactic or therapeutic treatment of a subject by dermal administration of an API, more specifically for the dermal administration of macromolecular API through pre-treated skin. By varying the pre-treatment of the skin, the dermal administration can be modified to achieve intradermal or transdermal delivery of the API.

The skin is an attractive organ for vaccination, treatment of allergy and immunotherapy. High abundance of dendritic cells, i.e. Langerhans cells, which capture antigens and migrate to lymph nodes, especially in the epidermis, where they trigger the differentiation of antigen-specific, naive T cells into effector T cells, are the key to a strong immune response. Thus, an allergen-specific immuno-cascade is mediated via antigen-presenting cells in the skin, which is important for successful sensitization. Hence, the dermal route opens up the possibility to lower the dose and improve the response rate as compared to other administration routes such as the subcutaneous or intramuscular route which are frequently used in the context of known vaccination regimens. Furthermore, therapeutic vaccination is a promising strategy against various cancers like hematological malignancies.

Hence in one preferred embodiment the pharmaceutical preparation of the invention is a vaccine preparation for the prophylactic or therapeutic treatment of a subject by dermal administration of the API, more specifically for the dermal administration of the API through pre-treated skin. The API is particularily a biopolymer; most preferred a protein or nucleic acid, most preferred a thermolabile protein or a thermolabile nucleic acid.

Accordingly, in one specific embodiment, the invention relates to the pharmaceutical preparation of the invention for use in vaccination, treatment of allergy or immunotherapy, in particular cancer immunotherapy.

A further aspect of the invention relates to a kit comprising the pharmaceutical preparation of the invention and means for the pre-treatment of the skin. Such means can be microneedles or a laser. In a particularily preferred embodiment the means for pre-treating the skin is a laser.

Yet another aspect of the invention relates to the manufacture of the pharmaceutical preparation of the invention. The manufacturing method of the invention comprises the following steps:
a. providing an API, in particular a macromolecule, most preferred a biopolymer such as a protein
b. providing a film for pharmaceutical use, optionally comprising an API
c. depositing the API on said film, in particular by printing
d. optionally repeating step c.

The water content of the film preferably does not exceed 15% (w/w) when the API is deposited. The API can be deposited onto the film either as a solid or as a liquid. If it is deposited as a liquid the composition comprising the API can either be identical with or different from the composition which is used for providing the film in a preceeding step.

The preferred mode of depositing the API is printing. The printing can be 3-D printing. In this embodiment the liquid composition comprising the API is used for its deposition onto the film. Thus the liquid composition serves as an "ink".

Other objects, features, advantages and aspects of the present invention will become apparent to those skilled in the art from the following description and appended claims. It should be understood, however, that the following description, appended claims, and specific examples, which indicate preferred embodiments of the application, are given by way of illustration only. Various changes and modifications within the essence and scope of the disclosed invention will become readily apparent to those skilled in the art from reading the following.

### DETAILED DESCRIPTION OF THE INVENTION

As used herein, the expressions defined herein are generally intended to preferably have the meanings as set forth below, except to the extent that the context in which they are used indicates otherwise.

The percentage values defined herein in general refer to weight-per-weight percentages unless otherwise indicated. In preferred embodiments, the values given herein encompass a range of ± 5 %, preferably of ± 2 % or ± 1 %.

The expression "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of".

The term "active pharmaceutical ingredient" (API) refers to a compound which exerts a pharmacological effect in a subject. An API is thus intended to furnish pharmacological activity or to otherwise have an effect in the diagnosis, cure, mitigation, treatment or prevention of disease, or to have effect in restoring, correcting or modifying physiological functions in a subject.

In preferred embodiments of the invention the API is a macromolecule. More preferably, the API is a biopolymer. Specifically, the API is a protein, peptide, peptide fragment, nucleic acid, nucleotide fragment, gene, aptamer, or any combination of the above.

More preferably, the API is selected from the group consisting of vaccines, allergens and systemic drugs. An allergen is an antigen capable of stimulating a type-I hypersensitivity reaction in atopic individuals through IgE responses. Preferably, the allergen is selected from the group of animal products, drugs, foods, insect stings, mold spores and plant pollens. The vaccine is preferably selected from the group consisting of a tumor-associated antigen, a self-antigen (e.g. an auto-antigen), a microbial antigen (e.g. a bacterial, viral or parasitic antigen), or an antigen comprising an immunorelevant epitope of any of the foregoing. Preferred examples of vaccines belong to the group of influenza vaccines. An example of a tumor-associated antigen is an antigenic peptide derived from HCV.

The term "antigen" as used herein refers to a molecule or a fragment thereof that is recognized by the subject's immune system. It may be presented by an antigen presenting cell (APC). Antigens are substances that can cause the immune system to produce an antibody response against it. Preferred antigens are macromolecules or molecules such as proteins, peptides, antibodies, polysaccharides, polynucleotides, RNA, DNA, lipids, glycosylated molecules, carbohydrates, organic or non-organic chemical compounds, naturally occurring modifications of such molecules, aptamers that are foreign to the host. Antigens comprise one or more immunologic epitopes.

The term "macromolecule" as used herein means an API having a molecular weight of at least 500, preferably of at least 750, 1,000, 1,500, 2,000, 2,500, 5,000, 10,000, 20,000, 50,000 or 100,000 Da. The macromolecule preferably does not have a molecular weight of more than 1,000,000 Da, 500,000 Da, more preferably not more than 400,000 Da, 300,000 Da, in particular not more than 200,000 Da. A macromolecule particularily preferred as a molecular weight between 500 and 1'000,000 Da, preferably between 1000 and 400,000 Da, most preferably between 10,000 and 200,000 Da.

A "biopolymer" within the context of the present invention refers to classes of polymeric molecules which can be produced by living organisms, regardless whether the particular polymeric molecule is actually produced by a living organism. Biopolymers include polynucleotides (RNA and DNA), polypeptides, proteins (summarized herein collectively as "protein" unless otherwise explictly outlined) and polysaccharides as well as products, fragments and modifications thereof. Biopolymers may be in an isolated form, as isolated and purified from an animal, plant, bacterial, viral or other source, or synthetically created or derived.

The pharmaceutical preparation of the invention preferably comprises at least one thermolabile API, in particular a thermolabile protein. The thermolabile API is preferably part of the film (either deposited on top of the film or being part of the composition forming the film by 3-D printing).

According to the present invention a protein API is "thermolabile" if it denaturates (reversibly or irreversibly) - i.e. partially or totally alters its native secondary, and/or tertiary, and/or quaternary structures - at 50°C resulting in a loss of at least 20%, more preferably at least 30%, more preferred at least 40%, even more preferred at least 50%, most preferred at least 70% of its bioactivity at ambient temperature.

"Thermostable" is a protein if it substantially maintains its biological function exhibited at ambient temperature even when heated to 50°C or more.

A nucleic acid is considered to be themolabile, if at least 20%, preferably at least 30%, more preferred at least 40%, even more preferred at least 50% of the nucleic acid is denaturated at 60°C (reversibly or irreversibly) compared to ambient temperature. In contrast, a nucleic acid is considered thermostable if it substantially maintains its native structure exhibited at ambient temperature when heated to 80°C.

The denaturing process of proteins and nucleic acids and the associated loss of activity and/or structure can be measured using techniques such as Dual-polarization interferometry, Circular dichroism (CD) spectroscopy, Quartz Crystal Microbalance with Dissipation monitoring (QCM-D) and Multi-Parametric Surface Plasmon Resonance (MP-SPR). For the assessment of protein thermostability also the thermal shift assay is known. Other assays, in particular such as ELISA, can also be used to study the structural/and or functional integrity of macromolecules, for example of proteins, in particular of vaccines and allergens.

"Deposition" according to the present invention is any form of application of material (here: the API or API containing composition) onto a film. The API can be deposited either in its pure form or contained in a composition. The deposited material can be a liquid or a solid ("liquid deposition" or "solid deposition"). The means of deposition are variable, for example pouring, dropping, spraying or printing, printing is preferred. Solid deposition typically requires that the API is solidified before deposition, preferably by lyophilization, i.e. freeze-drying the API. Preferably, according to the invention, the API is deposited by liquid deposition, most preferably by printing.

A "film" in the context of the present invention is a solid or semisolid carrier suited to bind, absorb, or otherwise receive the API. The film is preferably a layer, in particular a cohesive, solid or gelatinous layer. The film can be formed by casting or otherwise applying a composition containing a polymer which is suitable for forming a film solved or dispersed in a solvent and, optionally, further ingredients onto a surface. The film can also be produced by extrusion or printing.

Preferably, the film on the pharmaceutical preparation of the invention is dissolvable in water. Then, when applied onto the skin, the film at least partially can dissolve. Upon such at least partial dissolution of the film the API more easily migrates into the skin.

The film preferably is formed by polymers, in particular by hydrophilic polymers. The film in the pharmaceutical preparation of the invention thus preferably comprises at least one hydrophilic polymer. In a particularily preferred embodiment all polymers of the film are hydrophilc polymers.

The term "hydrophilic polymer" refers to a polymer or copolymers which contain(s) polar or charged functional groups, rendering them soluble in water. Hereinafter the term "polymer" collectively refers to polymers and copolymers unless otherwise explicitly mentioned.

In a preferred embodiment of the invention the hydrophilic polymer consists of at least 50% (w/w), even more preferred of at least 60% (w/w) and most preferred of at least 70% (w/w) of monomer residues which contain polar or charged groups.

In a preferred embodiment, the hydrophilic polymer is selected from the group consisting of polyvinyl pyrrolidone, polyvinyl alcohol, polyvinyl acetate, poly(vinylpyrrolidone-co-vinyl alcohol), polysaccharides, alginate, glycogen, starch and derivates thereof (such as maltodextrin), in particular amylose and amylopectin, pectin, chitin, callose, cellulose and derivatives thereof (such as hydroxymethylcellulose or hydroxypropylmethylcellulose) as well as combinations of two or more polymers or copolymers thereof.

In a more preferred embodiment the hydrophilic polymers are selected from the group consiting of polyvinyl alcohol, polysacchrides and cellulose (incuding derivates) or mixtures thereof. Even more preferred is the group consisting of polyvinyl alcohol, methylcellulose, hydroxymethylcellulose and hydroxypropylmethylcellulose and mixtures thereof.

In specifically preferred embodiments, the hydrophilic polymer is hydroxypropylmethylcellulose having an amount of hydroxypropyl of 5 to 15 %, an amount of methyl of 25 to 35 %, a viscosity of about 6 mPa*s as a 2% aqueous solution at 20°C. The amounts of hydroxypropyl and methyl refer to the relative amounts in relation to the monomeric units of the polymer. Thus, in a polymer comprising 10 % hydroxypropyl, 10 % or 1 out of 10 of the monomeric units of the polymer comprise a hydroxypropyl substituent.

Particular examples of preferred polymers are hydroxypropyl methylcellulose products which are commercially available, such as Pharmacoat 606 (hydroxypropyl methylcellulose, viscosity 6 mPa s, Shin-Etsu Chemical Co. Ltd., Japan), Methocel E3 and Methocel E5 (hydroxypropyl methylcellulose, viscosity 3 cps and 5 cps, respectively, Dow Chemical Company, USA).

In a preferred embodiment of the invention the hydrophilc polymer is present in the film in an amount of 50 to 75 %, preferably 55 to 65 %, more preferably 60 to 63 % (w/w) of the weight of the solids content of the base layer.

The film may further comprise a disintegration agent. A "disintegration agent" according to the invention is an agent which promotes or improves the dissolution or disintegration of the film. Disintegration agents in particular are hygroscopic agents and/or water-swellable agents. Preferably, disintegration agents are polymers, and in particular are selected from the group consisting of polyvinyl pyrrolidone, polyethylene glycol, croscarmellose sodium, sodium alginate and rice starch, sodium starch glycolate, Aerosil, hydrogen carbonates in combination with acids. A particularily preferred disintegration agent is polyvinyl pyrrolidone having an average particle size of less than 20 µm and/or an average nitrogen BET surface of more than 5 m²/g.

Particular examples of suitable disintegration agents are commercially available polyvinylpyrrolidones such as Kollidon CL-M and Kollidon CL-SF (BASF SE, Germany). The disintegration agent preferably is present in the film in an amount of 5 to 40 %, preferably 10 to 30 %, more preferably 15 to 25 % of the solids content of the film.

Furthermore, the film may comprise a softener. A "softener" according to the invention is an agent which increases the flexibility, plasticity or fluidity of the orodispersible film. The softener preferably is hydrophilic and/or water soluble and in particular is selected from the group consisting of glycerol, polyethylene glycol, propylene glycol and triethyl citrate, sorbitol, xylitol, 1,3-butandiole, isopropylpalmitate, dibutylsebacate, paraffin oil. A preferred example of the softener is glycerol. The softener preferably is present in the film in an amount of 5 to 40 %, preferably 10 to 30 %, more preferably 15 to 25 % of the solids content of the film.

The film may comprise further ingredients such as hydrophilizing agents like polyoxyethylenlaurylethers, such as commercially available Brij 35 (e.g. Merck KGaA, Germany), flavoring agents, odorants, aromatic agents and dyes.

The solvent of the composition forming the film preferably is water or a mixture comprising water.

In a particular embodiment, the film comprises or consists of 50 to 65 % hydroxypropylmethylcellulose, in particular hydroxypropyl-methylcellulose as defined above, 15 to 25 % polyvinyl pyrrolidone, in particular polyvinyl pyrrolidone as defined above, 14 to 22 % glycerol, and not more than 15 % water, even more preferred not more than 10, 8, 6, 4, 2, most preferred not more than 1.5 or 1 % (w/w) water.

The pharmaceutical preparation is suited for dermal administration. Dermal administration in general encompasses that the API remains on the skin, penetrates into the epidermis and/or dermis ("intradermal") or penetrates through the epidermis and dermis ("transdermal"). In the present invention intradermal and transdermal administraion is preferred.

The term "intradermal" administration herein refers to the delivery of the API to or into the epidermis and/or dermis. Thereby the API is delivered to the epidermal and/or dermal layer of the skin. The delivery is thus into the epidermis and/or the dermis, but preferably does not include the administration into subcutaneous layers of the skin. In particular, the intradermal administration includes the administration into the epidermis (intraepidermal) and/or the dermis, specifically into the basal layer (stratum basale), and/or into the basement membrane, which connects the epidermis to the dermis.

The expression "transdermal delivery" means delivery of the API through the skin and preferably into the blood circulation of the subject.

The intradermal route also allows a non-systemic, i.e. a local effect. This route is especially advantageous for vaccinations because the dendritic cells of the skin (Langerhans cells), which are responsible for the immunological response, reside in the epidermis. The transdermal route allows a systemic effect, because blood vessels are present in the subcutis below the dermis. The pharmaceutical preparation of the invention can hence be used to therapeutically treat local or systemic conditions. For the vaccination, in particular the non-systemic effect is important.

Preferably, the pharmaceutical preparation is a patch. Then, the pharmaceutical composition also comprises a backing. The pharmaceutical preparation may also be a transdermal therapeutic system (TTS). In this embodiment, the pharmaceutical preparation of the invention is used for transdermal administration of an API.

The term "subject" according to the invention means a human being, a nonhuman primate or another animal, in particular a mammal such as a cow, horse, pig, sheep, goat, dog, cat or a rodent such as a mouse and rat. In a particularly preferred embodiment, the subject is a human being.

If the API is deposited onto the film by liquid deposition, as outlined above, printing is particularily preferred. "Printing" means that the API is deposited in a liquid state (i.e. dissolved or suspended in water or any other solvent, optionally together with other components) and applied onto the film by a printer. Printing can be performed by flexographic, ink jet, piezoelectric or laser printing. Flexographic printing is particularly preferred. Also 3-D printing is possible.

The API can be printed in a liquid composition, which serves as an "ink". After being printed onto the film the liquid composition preferably also forms a film. Hence the pharmaceutical preparation of the invention preferably comprises at least a first film and a second film. Optionally, in a next step, further API can be deposited onto that first film. This step can be repeated. Preferably before depositing any further API, the film of the preceding step has a water content which does not exceed 15 % (w/w), preferably not 10, 8, 6, 4, 2, most preferred not 1.5 or 1 % (w/w).

The liquid composition (hereinafter also called printable composition or "ink") thus preferably contains at least one polymer which can form a film ("film forming polymer").

The film forming polymer of the liquid composition is pharmaceutically acceptable. It is preferably selected from the group consisting of hydroxypropylcellulose, hydroxyethylcellulose, crosslinked polyvinyl pyrollidone, ethyl cellulose, and poly methacrylates.

In preferred embodiments, the film-forming substance is hydroxypropylcellulose having a viscosity of about 5 to 7 mPa*s as a 2% aqueous solution at 25°C, and/or a weight-average molecular weight of about 70 to 90 kDa. In further preferred embodiments, the film-forming substance is hydroxypropylcellulose having a viscosity of about 10 to 12 mPa*s as a 2% aqueous solution at 25°C, and/or a weight-average molecular weight of about 120 to 160 kDa.

Particularly preferred examples of suitable film-forming substances for use in the printable composition are commercially available hydroxypropylcellulose products such as Klucel EX, Klucel EXF, Klucel JX and Klucel JXF (Ashland Aqualon Functional Ingredients, USA).

In the printable composition the film-forming substance preferably is present in an amount of 10 to 80 %, preferably 25 to 70 %, more preferably 30 to 55 % of the weight of the solids content of the composition. Preferably, the film-forming substance has a high solubility in organic solvents or mixture comprising organic solvents, wherein the mixture comprises less than 50%, preferably less than 20%, more preferably less than 10% water. The organic solvent preferably is an organic solvent, in particular a short-chain alcohol such as ethanol.

The printable composition may comprise further ingredients such as disintegration agent, in particular disintegration agents as defined above, softeners, in particular softeners as defined above, hydrophilizing agents like Brij 35, flavoring agents, odorants, aromatic agents, taste masking agents and colouring agents.

In a particular embodiment, the liquid composition comprises or consists of 25 to 75 % of the pharmaceutically active agent, 20 to 75 % hydroxypropylcellulose, in particular hydroxypropylcellulose as defined above, and less than 12 % ethanol.

Pharmaceutical preparation with a first film ("base layer") and a second film ("top layer"), having been manufactured by printing a liquid composition onto a first film, is described in detail in WO2013/023775 A1. This disclosure is fully incorporated herein by reference with respect to possible compositions and methods of manufacture of these films and the pharmaceutical preparations resulting therefrom.

In yet another preferred embodiment of the invention the "ink" is free from a film-forming polymer. In this embodiment the ink can be any aqueous solution or suspension comprising an API. This embodiment is exemplified with example 1 b) *infra.*

In a preferred embodiment the pharmaceutical preparation of the invention comprises a backing layer. A backing layer serves to protect the pharmaceutical preparation against the environment during the wear period. Backing layers are known to the person skilled in the art, e.g. from WO 2013/045420 A1 or WO 01/91718 A2. The film is directly or indirectly attached to the backing. Preferably the backing layer projects over the film. The backing layer can be a flexible or inflexible material. It can be made of polymeric or metal foils, such as aluminum foil, which may be used alone or coated with a polymeric substrate. Textile fabrics may also be used.

Preferably, the backing layer contains at least one pressure-sensitive adhesive. The adhesive facilitates adherence of the backing layer to the skin and/or preferably also to the film. Accordingly, in preferred embodiments, the backing layer adheres to the film and/or the skin. The pressure sensitive adhesive preferably is selected from the group consisting of polyisobutylenes, silicones, acrylates, polyurethanes, ethylene vinyl acetate, polystyrene block copolymers and derivatives as well as combinations of two or more polymers or copolymers thereof.

Most preferred, the backing layer is occlusive, i.e. it limits the diffusion of water vapor. A known method for the determination of water vapor permeability is the method according to DIN EN 13726-2. According to a preferred embodiment of the invention the water vapor permeability of the backing layer as measured according to DIN method EN 13726-2 (sample size of 20 cm² at 37°C and 18% relative humidity) does not exceed 300 g/(m² x 24h), more preferably it does not exceed 250, even more preferably does not exceed 200 and most preferred does not exceed 50 g/(m2 x 24h). Most preferred is a backing which is 100% occlusive, i.e. does not allow water vapor to pass.

The backing layer most preferably contains polymers from the group of polyisobutylenes, polyisoprene, polybutenes and styrene block polymers with isoprene or butadiene and copolymers and mixtures thereof. These polymers have water vapor barrier properties and are especially suitable as pressure sensitive adhesives when different types having different molecular weights are mixed. The relative amount of such polymers in the backing layer is preferably at least 20 % (w/w), more preferred at least 30, even more preferred at least 40 and most preferred at least 50% (w/w/). The most preferred polymer for the backing layer is polyisobutylene.

Furthermore, the backing layer preferably is impermeable to wound secretion and/or the API. The term "impermeable" herein refers to preventing or at least reducing the diffusion of a first material (the diffusing material) through a second material (the impermeable material). Impermeability can be determined by applying the diffusing material on one surface of the impermeable material and measuring the amount (volume or weight) of the diffusing material on the opposite side of the impermeable material after a certain time. Within the context of the invention, a material is impermeable, if not more than 20 % (w/w), preferably not more than 10 % (w/w), even more preferably not more than 5 % (w/w) diffusing material is measured in such test after 24 hours.

In preferred embodiments, the pharmaceutical preparation comprises a "protective layer". If a backing layer is present, the protective film is on the side opposite to the backing layer. Preferably, the protective layer projects beyond the film. The protective layer protects the pharmaceutical preparation, in particular the film, during storage and handling and is removed prior to the use of the pharmaceutical preparation.

The expression "residual water content" refers to the quantity of water contained in a material. Methods that determine water content include chemical titrations (for example the Karl Fischer titration), determining mass loss after freeze drying or the Dean-Stark method. Preferably, the material is dried in an oven set at 105 °C for 24 hours. Subtracting the weight after drying from the weight before drying gives the absolute amount of residual water contained in the material, from which the relative content can be calculated.

In preferred embodiments, the residual water content of the film does not exceed 15 % (w/w), preferably not 10, 8, 6, 4, 2 or even 1.5 or 1 % (w/w). In particular preferred embodiments, a pharmaceutical preparation, preferably a patch, is provided, which comprises an API printed to a film, wherein the film contains at least one hydrophilic polymer and the film has a residual water content below 2 %, in particular below 1.5 %, more particularly below 1 % (all values in w/w). Low residual water contents are particularly preferred, where it is intended to stabilize the API or to avoid the use of preservatives.

In preferred embodiments, the film has a surface area of 1 to 100 cm², in particular 2 to 50 cm², more particularly 5 to 25 cm². The API content of films of the preferred size preferably varies between 500 mg to 1 pg, preferred between 100 mg to 1 ng, most preferred between 50 mg to 1 µg. In a preferred embodiment, the film has a thickness of 0.001 to 5 mm, preferably of 0.01 to 2 mm, more preferably of 0.05 to 1 mm.

In preferred embodiments, the pharmaceutical preparation comprises a protective film. If a backing layer is present, the protective film is on the side opposite to the backing layer. Preferably, the protective film projects beyond the film. The protective film protects the pharmaceutical preparation, in particular the film, during storage and handling and is removed prior to the use of the pharmaceutical preparation at the subject treated therewith.

In a particular embodiment of the invention the pharmaceutical preparation is used for the dermal administration of API through pre-treated skin. The term "pre-treated skin" refers to skin, which has reduced barrier function compared with the untreated skin. "Pre-treatment" refers to any kind of skin treatment which leads to a reduction of the natural barrier function. Pre-treatment can be caused by any adequate method and is known to the skilled person. Such methods includes generating micropores by at least one microneedle, abrasion by chemicals (chemoablation), by lasers (laser ablation), by sonification, high energy radiation or by electricity (fulguration).

An overview about methods known to the skilled person for skin treatment, which are applicable as a pre-treatment according to the present invention provides the review of Loesch M.M. et al (Clin Cosmet Investig Dermatol. 2014; 7: 231-241. "Skin resurfacing procedures: new and emerging options").

"Pre-treatment" may thus be performed by "ablation" which refers to the removal of tissue, cells or components of the skin. Hence "ablated skin" denotes that portion of skin or skin tissue, which had been subjected to ablation. Ablation also includes "poration" (which has the same meaning as "microporation"). Poration means the formation of small channels, holes, micropores or pores to a desired depth in or through the skin of the subject. Preferably, the depth of the channels, holes, micropores or pores extends through the stratum corneum, in particular into the epidermis, or to the dermis. In some embodiments, the depth extends to the subcutis. In most preferred embodiments, the depth of the pores is between 30 µm and 120 µm.

In yet another aspect the invention relates to a kit comprising the pharmaceutical preparation of the invention and means for pre-treatment. The means for pre-treatment refers to a substance, composition or apparatus suited for pre-treatment, preferably suited for ablation, in particular microporation.

In a preferred embodiment the kit includes a means for the pre-treatment selecting from the group consisting of a laser instrument, at least one microneedle, an abrasion chemical and a generator of an electrical field. Within the context of the invention, a laser instrument, in particular a laser porator is preferred, in particular one, which is capable of adopting the laser setting as described *infra.* The laser instrument can preferably create a pore depth, pore diameter and geometric arrangement of pores in a matrix as described in the context of laser poration hereinafter.

In preferred embodiments, the laser instrument is portable, preferably a handheld laser instrument. In some embodiments, the kit comprises instructions for using the laser instrument in combination with the pharmaceutical preparation of the invention.

### Manufacturing of the pharmaceutical preparation of the invention

Yet another aspect of the invention relates to the manufacture of the pharmaceutical preparation of the invention. The manufacturing method of the invention comprises the following steps:
a. providing an API, in particular a macromolecule, most preferred a biopolymer such as a nucleic acid or a protein
b. providing a film for pharmaceutical use, optionally comprising an API
c. depositing the API on said film, in particular by printing
d. optionally repeating step c.

The solvent content (in particular the water content) of the film preferably does not exceed 15% (w/w) when the API is deposited. Even more preferred the solvent content does not exceed 10, 8, 6, 4, 2, most preferred not 1.5 or 1 % (w/w) of the film. If step d. is repeated - e.g. when the film is manufactured by 3-D printing - the deposition of the API (or the API containing composition; ("ink")) preferably requires that the film having been generated by the earlier steps also has a solvent (water) content which does not exceed 15% /w/w), more preferably 10, 8, 6, 4, 2, most preferred not 1.5 or 1 % (w/w).

Hence in one embodiment the invention relates to the manufacture of a film comprising the following steps:
a. providing an API, in particular a macromolecule, most preferred a biopolymer such as a nucleic acid or a protein
b. providing a film for pharmaceutical use, optionally comprising an API
c. depositing the API on said film, in particular by printing
d. optionally repeating step c,
wherein the film provided in step b. has a solvent (water) content which does not exceed 15% (w/w), more preferably 10, 8, 6, 4, 2, most preferred not 1.5 or 1 % (w/w).

If applicable, a drying step can be integrated between step b. and step c. If a dyring step is performed then preferably the drying temperature does not exceed ambient temperature, in particular it does not exceed 30 °C.

However, most preferred the method of the invention does not require drying of the film after the API has been deposited. Drying can simply occur while the printing process is ongoing. Hence, in a preferred embodiment no drying step is required after API depositon.

Thus in a preferred embodiment the invention relates to the manufacture of a film comprising the following steps:
a. providing an API, in particular a macromolecule, most preferred a biopolymer such as a nucleic acid or a protein
b. providing a film for pharmaceutical use, optionally comprising an API
c. depositing the API on said film by printing and concurrently drying the film
d. optionally repeating step c,
wherein, preferably, the film provided in step b. has a solvent (water) content which does not exceed 15% /w/w), more preferably 10, 8, 6, 4, 2, most preferred not 1.5 or 1 % (w/w).

The API can be deposited onto the film either as a solid or a liquid. If it is deposited as a liquid the composition comprising the API can either be identical with or different to the composition which is used for providing the film in a preceding step.

The preferred mode of depositing the API is printing. Printing of API by various methods is known to the skilled person (e.g. EP 2 741 734 B1 or DE 34 23 328 A1). Most preferred is flexographic printing. In this embodiment the liquid composition comprising the API is used for its deposition onto the film. Thus the liquid composition serves as an "ink". Printing can also be 3-D printing.

The API thus preferably is printed onto at least one surface of the film. The API is provided in a printable composition (for preferred embodiments cf. *supra).* The ink typically includes water. Optionally further solvents depending amongst others on the chemical properties of the API (e.g. hydrophilicity) can also be included. It may include buffer components and other additives, in particular additives that aid in the printing process or the adherence of the API to the film (as outlined in detail *supra).*

The API may also be lyophilized. Lyophilization usually requires a freeze-drying protocol specifically adapted to the API. The lyophilized API can be stabilized by excipients known to the skilled person. It can be applied either in a pure form or in a composition with excipients to at least one surface of the film.

A placebo film for deposition of the API can be produced by any method including solvent casting, extrusion and printing techniques. In preferred embodiments, the film is dried once it has been casted or extruded. Due to the absence of a thermolabile API drying can be achieved at elevated temperature. The film may comprise one or more thermostable APIs before the thermolabile API is deposited. The one or more thermostable APIs may be added to the film by any method including extrusion, solvent casting and printing.

### The therapeutic and prophylactic use of the pharmaceutical preparation of the invention

The invention further relates to the therapeutic and prophylactic use of the pharmaceutical preparations disclosed herein.

The pharmaceutical preparations of the invention and the pharmaceutical preparations produced by the manufacturing method of the invention, including the specific and preferred embodiments thereof, as well as the kits of the invention are particularly useful in therapy. The term "therapy" as used herein denotes treatment of one or more conditions. Treatment also includes prevention ("prophylactic treatment") and/or alleviation of one or more conditions. The term "condition" as used herein refers to disturbances in normal function or appearance and includes diseases, complications, disorders, syndromes, disabilities, stiffnesses, dysfunctions and/or symptoms thereof. In preferred embodiments, the pharmaceutical preparation of the invention is used in vaccination, treatment of allergy or immunotherapy, in particular cancer immunotherapy. It is also possible to use the pharmaceutical preparation in a skin prick test for the detection of allergies. Pricking the skin with a needle can be avoided if for example the skin is microporated by a laser.

Preferably, the pharmaceutical preparations of the invention are in particular used for the dermal administration, i.e. intradermal or transdermal delivery of the API, contained in the pharmaceutical preparation of the invention. It preferably comprises dermal administration of the pharmaceutical preparation of the invention to pre-treated skin. More specifically, a plurality of channels (e.g. micropores) can be produced at a predetermined site of the subject's skin by pre-treatment, preferably by laser poration; and the pharmaceutical preparation of the invention is dermally administered, i.e. topically applied, onto the microporated portion of the skin.

The channels created by pre-treatment improve the API to migrate from the pharmaceutical preparation through the channels and into the surrounding tissue. In a specific embodiment, the API migrates through the skin ("transdermal") into the blood circulation. Accordingly, the present invention permits intra- or transdermal delivery of APIs and in particular macromolecules.

Pre-treatment can be caused by any adequate method including generating micropores by at least one microneedle, abrasion by chemicals (chemoablation), by lasers (laser ablation), or by electricity (fulguration). For example, US 6,148,232 describes a technique for creating micro-channels by using an electrical field. Within the context of the invention laser ablation is preferred. A laser refers to a high-energy beam of light that can be directed into certain areas or tissues. The beams of a laser are preferably produced in one wavelength at a time and can vary in terms of the power or strength of the beam and the resulting tissue it can target. Laser ablation refers to an ablation method employing a laser porator for creating pores by a laser beam.

With laser ablation, any suitable wavelength can be used. Preferred wavelengths of lasers used in the context of the invention have a wavelength in which water has a high absorbance and in which structural or functional components of the cell have significantly less or even no absorbance. Accordingly, wavelengths typically include mid-infrared and higher wavelengths, and especially preferred wavelengths will be in the range of 2500 nm to 5000 nm. Most preferred laser wavelengths are at about 2500 nm to 3500 nm. For example, an Er:YAG laser has a wavelength of 2940 nm. The wavelength will preferably also be selected such that a minimum thermal destructive effect is achieved when the pulse time is 1 ms or less. According to WO 2016/169971, thermal tissue damage is minimized at a wavelength of about 3000 nm where the pulse time is less than 100 µs, and more typically about 10 µs.

To reduce the thermal damage of tissue surrounding a micropore to a minimum, the laser porator preferably creates a pulsed laser beam. In general, it is desirable to use relatively small laser pulse time/tissue exposure times. In the context of the invention, the laser pulse time/tissue exposure time is preferably less than 1 ms, more preferably less than 100 µs, even more preferably between 100 µs and 10 ns, and most preferably between 100 µs and 0.1 ps.

Particularly where small laser pulse time/tissue exposure times are used, multiple laser pulses onto the same pore will typically be required to form a micropore of desired depth.

It is further preferred that the irradiance is at least 104 W/cm², and more preferably at least 105 W/cm², even more preferably between 105 W/cm² and 109 W/cm², and most preferably between 105 W/cm² and 1012 W/cm² where energy doses of between about 0.01 J/cm² and 1000 J/cm², and more typically 0.1 J/cm² and 100 J/cm² are employed.

Ablative and non-ablative laser can be used. A non-ablative laser has a lower energy level than an ablative laser and tends to cause damage to subsurface areas of the targeted skin. An ablative laser has an intense energy that is used in bursts on the surface of the skin being treated. The intense energy heats the water within the surface layers of the skin, causing the water and tissue to vaporize. Each pass of the laser energy over the skin causes the outermost layers to be removed in a precise and controlled way to an appropriate depth of penetration. Preferred lasers are ablative.

It is further preferred that the laser is a fractional laser. Fractional lasers only damage certain zones within a selected targeted skin, producing a tiny dot or pixel-like area of treatment, hence only causing fractional damage from the heat of the light source. Fractional lasers can be ablative or non-ablative.

The laser used in most preferred embodiments of the invention includes an ablative fractional laser. For example, in some embodiments of the present invention, a fractional ablative Erbium-YAG (Er:YAG) laser is used, such as the Sciton Profractional XC having a spot size of 430 µm, a depth range 20-1500 µm, relative densities 11% to 22%. In other embodiments of the present invention, a Lumenis Ultrapulse laser, using a 10,600 nm wavelength and 240 watts, 225 mJ of energy, spot size of 120 µm, depth ranges from 75-1500 µm is used. In preferred embodiment, the laser used in context of the present invention is the fractional ablative laser poration system P.L.E.A.S.E.® (Precise Laser Epidermal System) available from Pantec Biosolutions AG (Rugell, Liechtenstein). This laser system features a fractional Er:YAG laser.

With laser ablation it is possible to reproducibly produce a plurality of small channels, e.g. pores, in the skin (termed microporous or microporated skin herein). Variation of laser settings allows for precisely adjusting pore depth, pore diameter and geometric arrangement of pores in a matrix.

The micropore depth preferably depends on the type of API, in particular whether a local or systemic effect is to be achieved and whether it is desirable to deliver the API to the dendritic cell-rich skin, i.e. to the epidermis of the skin.

Preferably, the micropores are deep enough to create an injury response. An injury response within the context of the invention is a natural, innate, or endogenous response of the body as a result of laser treatment. Preferably, it includes an inflammatory response. It may include initiating a cytokine cascade whereby cytokines, immune cells, and other factors are recruited to the site of laser injury. The injury response may occur at any point of the skin which is damaged by the laser light, preferably along the entire length of the micropores or at least a part thereof. In preferred embodiments, the micropore depth reaches into the epidermis, dermis or subcutis, preferably into the epidermis or dermis. In most preferred embodiments, the depth is between 10 µm and 150 µm.

The average diameter of the micropores is preferably 10 to 2000 µm, more preferably 50 to 1000 µm, most preferably 100 to 500 µm.

Preferably, a plurality of micropores are arranged in a matrix. This can be achieved by passing the laser in a scanning pattern over the targeted skin, thus creating a plurality, or matrix, of micropores therein. The matrix has preferably a circular, rectangular or squarish shape.

The space between the micropores and/or the ratio of ablated tissue corresponding to micropores and intervening non-lasered skin defines the micropore density. The matrix has preferably a surface area that is equal to or, preferably, smaller than the surface area of the film. Accordingly, the matrix surface area is equal to or, preferably less than 1 to 100 cm2, in particular 2 to 50 cm², more particularly 5 to 25 cm². The number of micropores per matrix surface area (i.e. density) is thus between 1 to 1,000 per cm², preferably 5 to 1,000 per cm², more preferably 10 to 1,000 per cm².

Once pre-treatment is done and at least one micropore is formed in the skin, the pharmaceutical preparation of the invention is applied to the pre-treated portion of the skin. Preferably, the pharmaceutical preparation is placed on the subject's skin such that the film completely covers the microporated portion of the skin. The subject's innate and endogenous mechanisms enable the API to enter the skin through the channels created by pre-treatment.

A good therapeutic effect is achieved when the pharmaceutical preparation is applied immediately after pre-treatment, preferably within 1 hour, more preferably within 30 minutes, most preferably within 10 minutes, in particular within 5 or 2 minutes.

The duration of effective treatment is limited by the subject's endogenous mechanisms to heal the pre-treated skin and restore its barrier function. After 12 to 48 hours the channels may be no longer accessible for the APIs. Hence, it is preferred that the pharmaceutical preparation is applied for a period of 1 to 48 hours, preferably 2 to 36 hours, more preferably 6 to 24 hours to the pre-treated skin.

Though the pharmaceutical preparation of the invention may be administered by a single administration to achieve the desired therapeutic effect, such as immune response, specific embodiments refer to repeated administration. For example, the pharmaceutical preparation of the invention may be administered as a first dose followed by one or more booster doses, within a certain timeframe. A preferred vaccination or administration regimen encompasses administration of three doses, e.g. a first dose (e.g. on day 0), a second dose (e.g. on day 5-40), and a third dose (e.g. on day 10-100) using a first, second and third pharmaceutical preparation of the invention, respectively.

In a preferred embodiment, the skin is pretreated by a laser and the pharmaceutical preparation comprises a protein or nucleic acid greater than 500 Da as API that is printed onto the film and is used in the prophylactic or therapeutic treatment of a subject by intradermal administration. The pharmaceutical preparation preferably as an occlusive backing.

In another preferred embodiment, the skin is pretreated by a laser and the pharmaceutical preparation comprises a protein between 10,000 Da and 200,000 Da as API that is printed onto the film and is used in the prophylactic or therapeutic treatment of a subject by intradermal administration. The pharmaceutical preparation preferably as an occlusive backing.

In another preferred embodiment, the skin is pretreated by a laser and the pharmaceutical preparation comprises a vaccine as API that is printed onto the film and is used in the prophylactic or therapeutic treatment of a subject by intradermal administration.

### SUMMARY OF THE FIGURES

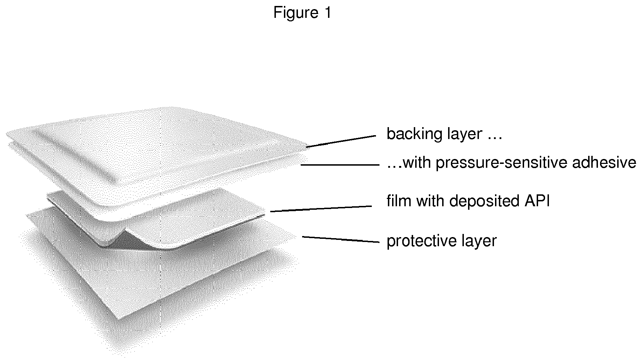

- Fig. 1:: Pharmaceutical composition according to one embodiment of the present invention. This embodiment of the invention is especially preferred.
- Fig. 2:: Sketch of the used laser according to one embodiment of the present invention
- Fig. 3:: Visualization of the laser-induced microchannels in human skin by micro-CT
- Fig. 4:: *Ex-vivo* TEWL measurements after laser treatment
- Fig. 5:: *In-vivo* TEWL measurements after laser treatment
- Fig. 6:: *In-vivo* TEWL measurements after laser treatment with and without occlusion by plaster
- Fig. 7:: *In-vivo* TEWL measurements after laser treatment with and without occlusion by a transdermal therapeutic system (TTS)
- Fig. 8:: Permeation test (A: film freshly applied; B: films after 24h on the skin (left treated, right untreated))
- Fig. 9:: Horizontal permeation cell
- Fig. 10:: Solutions and hydrogel patches, perforation densities 1, 3 and 6
- Fig. 11:: Solutions and PVA-patches, perforation densities 3 and 12
- Fig. 12:: Hydrophilic polymer patches, perforation density 15
- Fig. 13:: Hydrophilic polymer patches comprising dextran, perforation density 15

### EXAMPLES

### Example 1: Manufacturing of a film according to the invention

### a) re. printable composition ("ink') containing a film-forming substance

The ingredients of a placebo film were Pharmacoat^{®} 606 (hydroxypropyl methylcellulose, Shin-Etsu Chemical Co., Japan) or Methocel^{®} E5 (hydroxypropyl methylcellulose, Dow Chemical Comp., USA), Kollidon® CLM (polyvinylpyrrolidone, BASF SE, Germany), Glycerol and water. The film was casted onto an intermediate liner using a coating machine equipped with a comma blade and conveyed through an oven with four heating-zones. The film was rolled up to a jumbo roll and cut into daughter rolls with a width of 2 cm and a length up to 100 m.

The ingredients of printable composition the ink were Klucel® EX (hydroxypropylcellulose, Ashland, USA), brilliant blue and ethanol. Any API can be included.

Flexography was used as printing method. The machine was equipped with an anilox roller with a capacity of 11.71 cm³/m² or 80 cm³/m². Each film was printed for four times with a printing speed of 16 m/min. Final film size was 2 cm to 3 cm.

### b) re. printable composition ("ink') without a film-forming substance

A placebo film (80 g/cm²) was prepared with partially hydrolyzed polyvinyl alcohols of low viscosity (eg, Mowiol® 4-88 or Mowiol® 8-88) and then cooled to room temperature. Pieces of 2 x 2 cm² were punched out.

A liquid composition ("ink") containing sodium chloride, potassium, chloride disodium phosphate dihydrate, potassium dihydrogen phosphate and water for injections and inactivated influenca virus as antigen was deposited onto the film by flexographic printing (5 replications).

The pharmaceutical preparation according to the invention furthermore comprised a backing layer includin a mono- or bielastic flexible polyester backing (KOB051 or KOB053) and polyisobutylene (henke Durotak 87-6908 or Durotak 87-618A). The backing had a size exceeding the film, namely 4 x 4 cm² The coating weight was 30 g/m².

### Example 2: Manufacturing of hydrogel patches

Hydrogel patches were prepared according to DE 102010038312 A1. 40 g aqueous buffer were provided. Carbopol and glycerin were dispersed. Sodium hydroxide was dissolved in the remaining water and agar agar was dispersed in this solution. The two suspensions were then mixed. After a homogeneous gel had formed, the API was added and stirred in the matrix for 3 hours.

### Example 3: Manufacturing of films

### Film forming compositions

*Mowiol® 4-88 and Mowiol® 8-88 (polyvinyl alcohol)* as *hydrophilic polymer* Partially hydrolyzed polyvinyl alcohols of low viscosity (eg, Mowiol® 4-88 or Mowiol® 8-88) were dissolved in hot water and then cooled to room temperature. The API was dissolved in an aqueous solution and added to the viscous mass.

*Pullulan (polysaccharide polymer consisting of maltotriose units)* as *hydrophilic polymer* The water soluble linear polysaccharide Pullulan was dissolved in an aqueous solution. After a homogeneous mass had formed, the API was added and stirred in the matrix for 3 hours.

*Metolose® 60SH (methyl cellulose and (hydroxypropyl)methyl cellulose)* / *Pharmacoat® 605 (hydroxypropylmethyl cellulose*) *(1:1)* as *hydrophilic polymer* Medium-viscose methyl celluloses (e.g. Metolose® 60SH and Pharmacoat® 605 1:1) were dissolved in an aqueous solution. After a homogeneous mass had formed, the API was added and stirred in the matrix for 3 hours.

### Film preparation

The adhesive API-containing matrices were poured onto a liner with a doctor blade, the Quadruple Film Applicator from ERICHSEN GmbH & Co (Hemer, Germany). The films were dried at 27°C for 45 min. After drying, the protective film was laminated.

### Example 4: Pre-Treatment of ex-vivo human skin with a laser

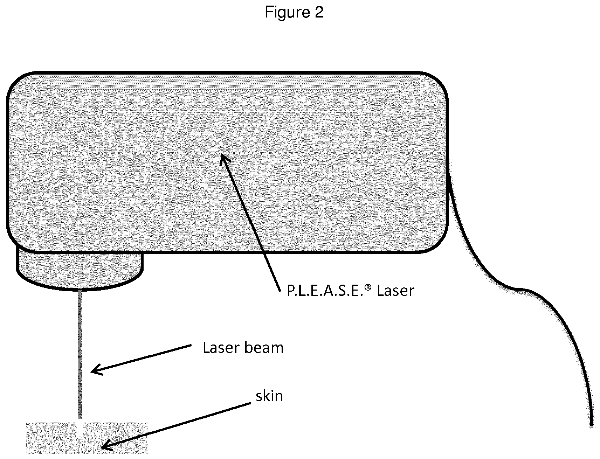

*Ex-vivo* human skin was treated with a P.L.E.A.S.E.® Laser (Pantec Biosolutions) (Fig. 2). Different perforation densities (for example, 15, 50 and 100 microchannels per 0.82 cm²) and different penetration depths (for example, 59 µm) of the laser can be set as needed.

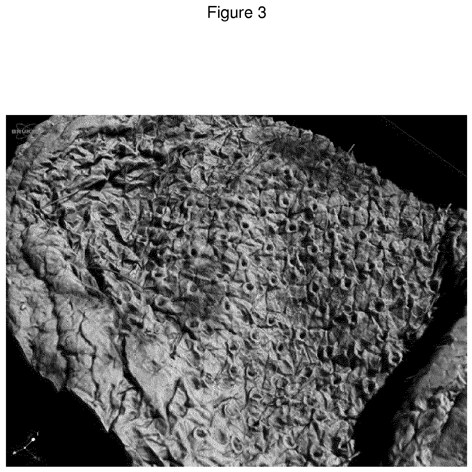

For imaging the skin after perforation, SKYSCAN micro-computed tomography (micro-CT) was used. The microchannels created with the P.L.E.A.S.E. laser were not only evenly distributed but also extremely homogenous and reproducible in pore dimensions (diameter and depth) as shown in Fig. 3.

### Example 5: Transepidermal water loss

Transepidermal water loss is presumably and among other factors responsible for partly or fully dissolving the pharmaceutical preparation.

*In-vivo* and *ex-vivo* transepidermal water loss (TEWL) was measured using a Tewameter® TM 300 w (Courage + Khazaka electronic GmbH).

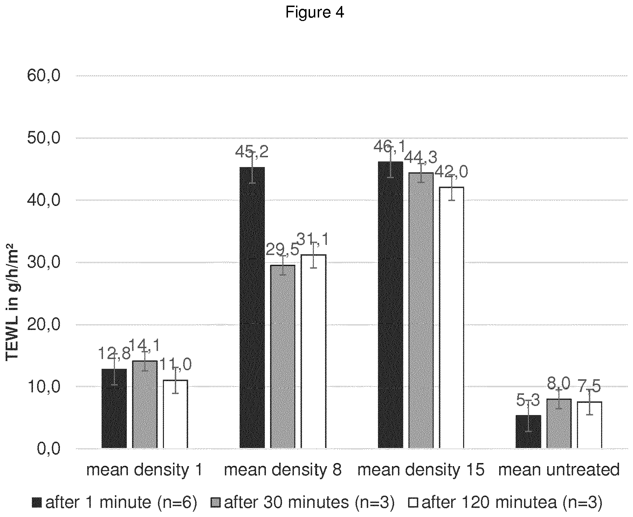

*Ex-vivo* TEWL measurements show significant increase of the TEWL with an increasing amount of microchannels per skin area (Fig. 4).

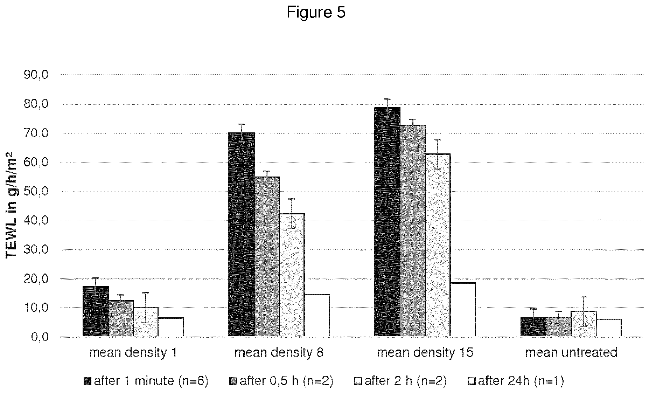

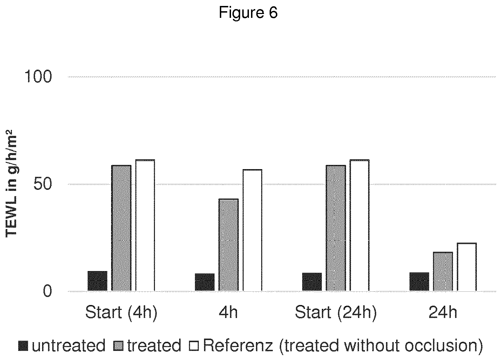

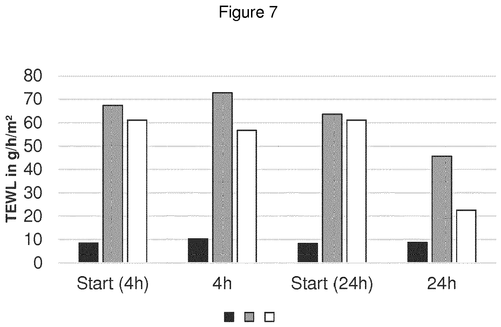

*In-vivo* TEWL measurements also show a significant increase of the TEWL with an increasing amount of microchannels per skin area and fast skin regeneration (Fig. 5) even under occlusive conditions (Fig. 6 and Fig. 7.)

Under occlusion with a TTS, the pores stay open (slower healing) so that more water is released from the pores.

### Example 6: Permeation test

Patches comprising polyvinyl alcohol (PVA) containing 1% (w/w) Trypan Blue were prepared by solvent casting on an occlusive backing layer with a thickness of 500 µm using a Film Applicator.

*Ex-vivo* human skin was treated with a P.L.E.A.S.E.® Laser (Pantec Biosolutions) to result in the perforation density of 15 per 0.82 cm². The penetration depth of the laser was set to 59 µm.

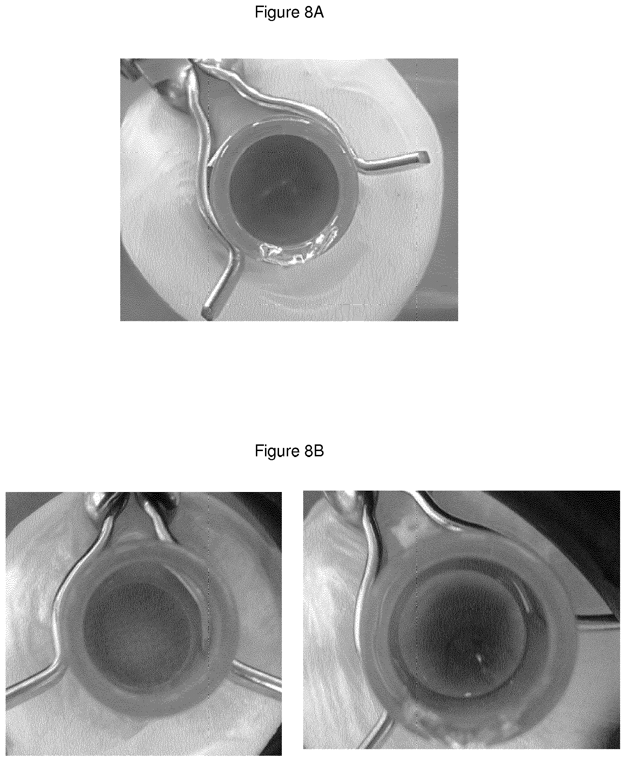

Film freshly applied is shown in Fig. 8A. After 24 h at 32°C in the permeation model, in case of treated skin only the microchannels are colored by trypan blue (Fig. 8B left). In case of untreated skin, the film is resolved at the edges by the water permeability of the skin but still remained fully colored indicating no permeation of the color across the skin (Fig. 8B right).

Thus, pre-treatment of the skin promotes permeation of the blue color into the microchannels.

### Example 7: Skin permeation tests with pharmaceutical preparations manufactured by solvent casting

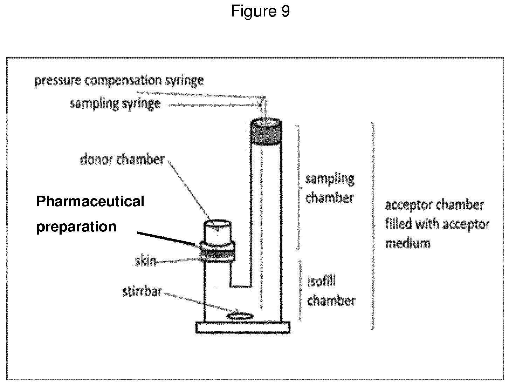

*Ex-vivo* Franz-cell skin permeation was performed under sink conditions to examine whether the API passes through the perforated skin. A horizontal permeation cell (Fig. 9) was used.

The determination of the API was done by HPLC with fluorescence-detection.

The solution or patch (pharmaceutical preparation manufactured by solvent casting) was placed on untreated and microporated *ex-vivo* human skin and the flux of the API across skin was investigated over a time period of 48 hours.

### a) Solutions and hydrogel patches, perforation densities 1, 3 and 6

Hydrogel patches containing 5% (w/w) desmopressin-acetate were prepared as described in DE 102010038312 A1 utilizing the classical solvent casting process with a wet coating weight of 2250 µm (see also *supra).* They were compared with aqueous solutions containing 5% (w/w) desmopressin-acteate.

*Ex-vivo* human skin was treated with a P.L.E.A.S.E.® Laser (Pantec Biosolutions) to result in three different perforation densities 1, 3 and 6 (15, 50 and 100 microchannels per 0.82 cm²). The penetration depth of the laser was set to 59 µm.

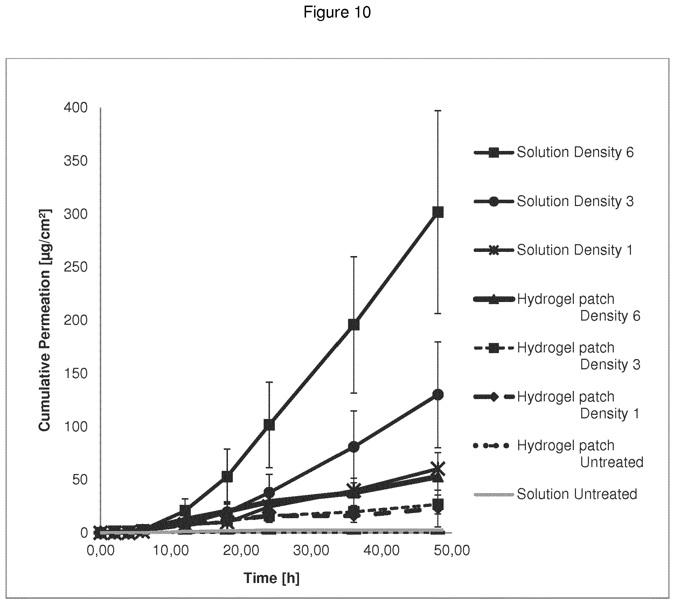

The result is shown in Fig. 10. Significant increase of the flux rate and the cumulative permeated amount of desmopressin with an increasing amount of microchannels per skin area was observed. Already with 50 microchannels the cumulative permeated amount of desmopressin from the hydrogel patch after 24 hours (ca. 16 µg/cm²) significantly exceeded the recommended daily dose of Minirin® (4 µg).

Thus, large macromolecules readily diffuse from an aqueous solution or hydrophilic patch and can be efficiently delivered through microporated skin.

### b) Solutions and PVA-patches, perforation densities 3 and 12

Patches comprising polyvinyl alcohol (Mowiol® 8-88) containing 5% (w/w) of desmopressin-acetate were prepared by solvent casting on an occlusive backing layer with a thickness of 500 µm using a Film Applicator (see also *supra).* They were compared with aqueous solutions containing 5% (w/w) of desmopressin-acetate.

*Ex-vivo* human skin was treated with a P.L.E.A.S.E.® Laser (Pantec Biosolutions) to result in the perforation densities 3 and 12 per 0.82 cm². The penetration depth of the laser was set to 59 µm.

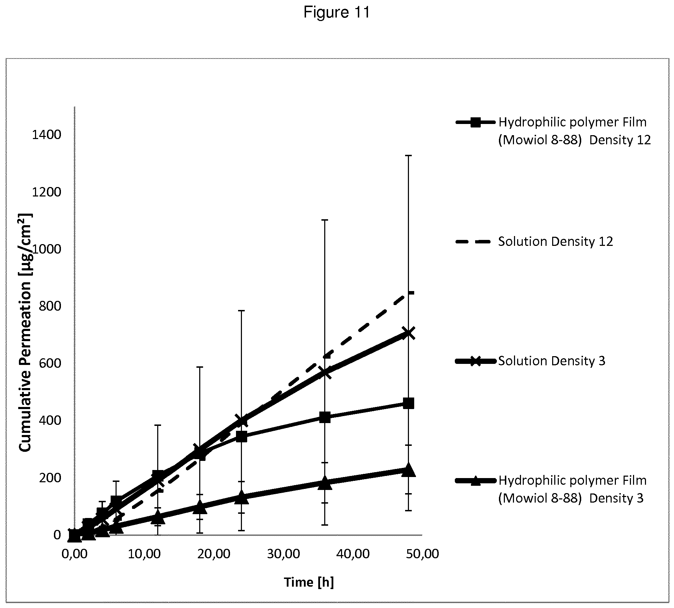

The result is shown in Fig. 11. Also Hydrophilic polymer films exceed the recommended daily dose of Minirin® (4 µg) significantly.

It has thus been shown that dry hydrophilic films can be used to efficiently deliver large macromolecules through microporated skin.

### c) Hydrophilic polymer patches, perforation density 15

Hydrophilic polymer films containing 5% (w/w) of desmopressin-acetate were manufactured by solvent casting on an occlusive backing layer with a thickness of 500 µm using a Film Applicator (see also *supra).* The polymers used were Metolose® 60SH (methyl cellulose and (hydroxypropyl)methyl cellulose) / Pharmacoat® (hydroxypropylmethyl cellulose) (1:1), Mowiol® 8-88 (polyvinyl alcohol), Pullulan (polysaccharide polymer consisting of maltotriose units) and Mowiol® 4-88 (polyvinyl alcohol).

*Ex-vivo* human skin was treated with a P.L.E.A.S.E.® Laser (Pantec Biosolutions) to result in the density of 15 per 0.82 cm². The penetration depth of the laser was set to 59 µm.

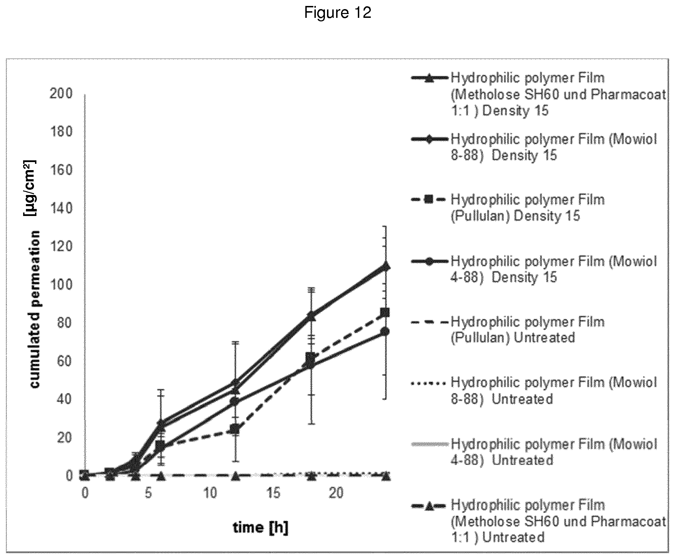

The result is shown in Fig. 12. No permeation of the ca. 1000 g/mol peptide was found on intact skin ("untreated"). Over the investigated time period of 24 hours up to 110 µg/cm² of desmopressin permeated through the microporated skin (which is more than 25 times of the recommended daily dose). All polymer compositions were very efficient.

### d) PVA-patches comprising dextran, perforation density 15

Patches comprising polyvinyl alcohol and dextran (10% (w/w) FITC dextran; 100,000 and 250,000 g/mol dextran) were prepared.

*Ex-vivo* human skin was treated with a P.L.E.A.S.E.® Laser (Pantec Biosolutions) to result in the density of 15 per 0.82 cm². The penetration depth of the laser was set to 59 µm.

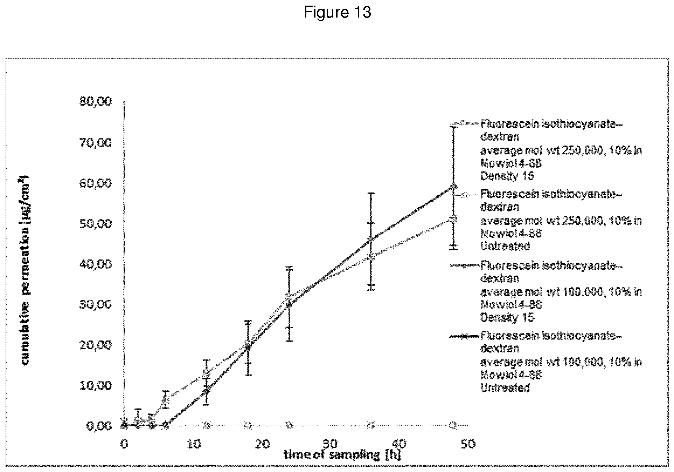

The result is shown in Fig. 13. Over the investigated time period of 48 hours up to 60 µg/cm² permeated through the microporated skin. The smaller dextran (100,000 g/mol) showed a slightly better permeation. No permeation was detected through the untreated skin.

## Claims

1. A pharmaceutical preparation comprising an active pharmaceutical ingredient (API) and a film for use in the prophylactic or therapeutic treatment of a subject by dermal administration, wherein the API is deposited onto said film.

2. The pharmaceutical preparation according to any of the preceding claims, wherein the API has a molecular weight of at least 500 Da, preferably of between 500 Da and 500,000 Da, more preferably between 10,000 Da and 200,000 Da.

3. The pharmaceutical preparation according to claim 1 or 2, wherein the API is a protein or nucleic acid.

4. The pharmaceutical preparation according to any of the preceeding claims, wherein the API is deposited onto the film by printing.

5. The pharmaceutical preparation according to any of the preceeding claims, wherein the water content of the film does not exceed 15 % (w/w), preferably not 10, 8, 6, 4, 2, most preferred not 1.5 or 1 % (w/w).

6. The pharmaceutical preparation according to any of the preceeding claims furthermore including a backing layer, preferably an occlusive backing layer.

7. The pharmaceutical preparation according to any of the preceeding claims, wherein the water vapor permeability of the backing layer does not exceed 300 g/(m² x 24h).

8. The pharmaceutical preparation according to any of the claims 5 or 6, wherein the backing layer contains at least one polymer selected from the group consisting polyisobutylene, polyisoprene, polybutene and styrene block polymer with isoprene or butadiene and copolymers and mixtures thereof.

9. The pharmaceutical preparation according to any of the preceding claims, wherein the film comprises at least one hydrophilic polymer, preferably selected from the group consisting of polyvinyl pyrrolidone, polyvinyl alcohol, polyvinyl acetate, poly (vinylpyrrolidone-co-vinyl alcohol), polysaccharides, alginate, glycogen, starch, in particular amylose or amylopectin, pectin, chitin, callose, cellulose and derivatives as well as combinations of two or more polymers or copolymers thereof, in particular cellulose and derivatives.

10. The pharmaceutical preparation according any of the preceeding claims, wherein the pharmaceutical preparation is dermally administered through at least one micropore.

11. The pharmaceutical preparation according to claim 5, wherein the at least one micropore is generated by a laser instrument or by at least one microneedle, preferably by a laser.

12. The pharmaceutical preparation according to any of the preceding claims for use in vaccination, treatment of allergy or immunotherapy, in particular cancer immunotherapy.

13. A method for manufacturing the pharmaceutical preparation according to any of the preceding claims comprising the following steps:
a. providing an API, in particular a macromolecule, most preferred a biopolymer such as a nucleic acid or a protein
b. providing a film for pharmaceutical use, optionally comprising an API
c. depositing the API on said film, in particular by printing
d. optionally repeating step c.

14. The method of claim 13, wherein the film provided in step b. has a water content which does not exceed 15% /w/w), more preferably 10, 8, 6, 4, 2, most preferred not 1.5 or 1 % (w/w).

15. A kit comprising the pharmaceutical preparation as defined in any of the preceding claims and means for generating micropores, preferably a laser instrument.
